(19) Europäisches Patentamt
European Patent Office
Office européen des brevets

(11) Publication number : 0 686 629 A2

# (12) EUROPEAN PATENT APPLICATION

(21) Application number : 95303818.9

(22) Date of filing : 05.06.95

(51) Int. Cl.[6] : C07D 209/14, C07D 209/18, C07D 307/81, C07D 307/84, C07D 333/68, C07D 215/48, C07D 401/12, C07D 405/12, C07D 409/12, A61K 31/33

(30) Priority : 10.06.94 US 259266

(43) Date of publication of application : 13.12.95 Bulletin 95/50

(84) Designated Contracting States : AT BE CH DE DK ES FR GB GR IE IT LI LU NL PT SE

(71) Applicant : ELI LILLY AND COMPANY
Lilly Corporate Center
Indianapolis Indiana 46285 (US)

(72) Inventor : Hipskind, Philip Arthur
3660 South Farmstone Circle
New Palestine, Indiana 46163 (US)

(74) Representative : Tapping, Kenneth George et al
Lilly Industries Limited
European Patent Operations
Erl Wood Manor
Windlesham Surrey GU20 6PH (GB)

## (54) Cyclohexyl tachykinine receptor antagonists

(57) This invention provides a series of substituted cyclohexanes which are useful in the treatment or prevention of a physiological disorder associated with an excess of tachykinins. This invention also provides methods for the treatment of such physiological disorders as well as pharmaceutical formulations which employ these novel compounds.

EP 0 686 629 A2

Tachykinins are a family of peptides which share the common amidated carboxy terminal sequence,

Phe-Xaa-Gly-Leu-Met-$NH_2$

hereinafter referred to as SEQ ID NO:1. Substance P was the first peptide of this family to be isolated, although its purification and the determination of its primary sequence did not occur until the early 1970's. Substance P has the following amino acid sequence,

Arg-Pro-Lys-Pro-Gln-Gln-Phe-Phe-Gly-Leu-Met-$NH_2$

hereinafter referred to as SEQ ID NO:2.

Between 1983 and 1984 several groups reported the isolation of two novel mammalian tachykinins, now termed neurokinin A (also known as substance K, neuromedin L, and neurokinin α), and neurokinin B (also known as neuromedin K and neurokinin β). See, J.E. Maggio, Peptides, 6 (Supplement 3):237-243 (1985) for a review of these discoveries. Neurokinin A has the following amino acid sequence,

His-Lys-Thr-Asp-Ser-Phe-Val-Gly-Leu-Met-$NH_2$

hereinafter referred to as SEQ ID NO:3. The structure of neurokinin B is the amino acid sequence,

Asp-Met-His-Asp-Phe-Phe-Val-Gly-Leu-Met-$NH_2$

hereinafter referred to as SEQ ID NO:4.

Tachykinins are widely distributed in both the central and peripheral nervous systems, are released from nerves, and exert a variety of biological actions, which, in most cases, depend upon activation of specific receptors expressed on the membrane of target cells. Tachykinins are also produced by a number of non-neural tissues.

The mammalian tachykinins substance P, neurokinin A, and neurokinin B act through three major receptor subtypes, denoted as NK-1, NK-2, and NK-3, respectively. These receptors are present in a variety of organs.

Substance P is believed inter alia to be involved in the neurotransmission of pain sensations, including the pain associated with migraine headaches and with arthritis. These peptides have also been implicated in gastrointestinal disorders and diseases of the gastrointestinal tract such as inflammatory bowel disease. Tachykinins have also been implicated as playing a role in numerous other maladies, as discussed infra.

In view of the wide number of clinical maladies associated with an excess of tachykinins, the development of tachykinin receptor antagonists will serve to control these clinical conditions. The earliest tachykinin receptor antagonists were peptide derivatives. These antagonists proved to be of limited pharmaceutical utility because of their metabolic instability.

Recent publications have described novel classes of non-peptidyl tachykinin receptor antagonists which generally have greater oral bioavailability and metabolic stability than the earlier classes of tachykinin receptor antagonists. Examples of such newer non-peptidyl tachykinin receptor antagonists are found in United States Patent 5,328,927, issued July 12, 1994; United States Patent 5,360,820, issued November 1, 1994; United States Patent 5,344,830, issued September 6, 1994; United States Patent 5,331,089, issued July 19, 1994; European Patent Publication 591,040 A1, published April 6, 1994; Patent Cooperation Treaty publication WO 94/01402, published January 20, 1994; Patent Cooperation Treaty publication WO 94/04494, published March 3, 1994; and Patent Cooperation Treaty publication WO 93/011609, published January 21, 1993.

In essence, this invention provides a class of potent non-peptide tachykinin receptor antagonists. By virtue of their non-peptide nature, the compounds of the present invention do not suffer from the shortcomings, in terms of metabolic instability, of known peptide-based tachykinin receptor antagonists.

This invention encompasses methods for the treatment or prevention of a physiological disorder associated with an excess of tachykinins, which method comprises administering to a mammal in need of said treatment an effective amount of a compound of Formula I

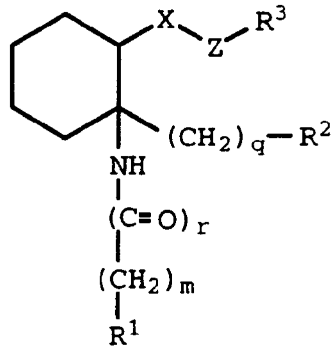

I

wherein:

X is -NH-, -O-, or $-S(O)_p-$,

where p is 0-2;

Z is $-(CHR^4)_j-(CHR^6)_k-$, where,

j is 0 or 1;

k is 0 or 1; and

$R^4$ and $R^6$ are independently selected from the group consisting of hydrogen and $C_1$-$C_3$ alkyl;

m and q are independently 0-3;

r is 0 or 1;

$R^3$ is phenyl, phenyl-($C_1$-$C_6$ alkylidenyl)-, $C_3$-$C_8$ cycloalkyl, $C_5$-$C_8$ cycloalkenyl, $C_1$-$C_8$ alkyl, naphthyl, $C_2$-$C_8$ alkenyl, or hydrogen;

any one of which groups except hydrogen may be substituted with one or two moieties independently selected from the group consisting of halo, $C_1$-$C_3$ alkoxy, $C_1$-$C_3$ alkylthio, nitro, trifluoromethyl, and $C_1$-$C_3$ alkyl; and

$R^2$ is phenyl, 3-indolyl, 2-indolyl, 3-benzothienyl, 3-benzofuranyl, 3-benzofuranyl, or naphthyl, said groups being optionally substituted with one to three moieties independently selected from the group consisting of $C_1$-$C_6$ alkyl, $C_1$-$C_6$ alkoxy, halo, or trifluoromethyl;

$R^1$ is trityl, phenyl, diphenylmethyl, phenoxy, phenylthio, hexamethyleneiminyl, piperazinyl, piperidinyl, pyrrolidinyl, morpholinyl, indolinyl, indolyl, benzothienyl, benzofuranyl, quinolinyl, isoquinolinyl, tetrahydropyridinyl, reduced quinolinyl, reduced isoquinolinyl, phenyl-($C_1$-$C_6$ alkylidenyl)-, phenyl-($C_1$-$C_4$ alkoxy)-, quinolinyl-($C_1$-$C_6$ alkylidenyl)-, isoquinolinyl-($C_1$-$C_6$ alkylidenyl)-, reduced quinolinyl-($C_1$-$C_6$ alkylidenyl)-, reduced isoquinolinyl-($C_1$-$C_6$ alkylidenyl)-, benzoyl-($C_1$-$C_6$ alkylidenyl)-, $C_1$-$C_4$ alkyl, or $-NH-CH_2-R^5$;

any one of which $R^1$ groups may be substituted with halo, $C_1$-$C_4$ alkyl, $C_1$-$C_4$ alkoxy, trifluoromethyl, amino, $C_1$-$C_4$ alkylamino, or di($C_1$-$C_4$ alkyl)amino;

or any one of which $R^1$ groups may be substituted with phenyl, piperazinyl, $C_3$-$C_8$ cycloalkyl, benzyl, $C_1$-$C_4$ alkyl, piperidinyl, pyridinyl, pyrimidinyl, $C_2$-$C_6$ alkanoylamino, pyrrolidinyl, $C_2$-$C_6$ alkanoyl, or $C_1$-$C_4$ alkoxycarbonyl;

any one of which groups may be substituted with halo, $C_1$-$C_4$ alkyl, $C_1$-$C_4$ alkoxy, trifluoromethyl, amino, $C_1$-$C_4$ alkylamino, di($C_1$-$C_4$ alkyl)amino, or $C_2$-$C_4$ alkanoylamino; and

$R^5$ is pyridyl, anilino-($C_1$-$C_6$ alkylidenyl)-, or anilinocarbonyl;

or a pharmaceutically acceptable salt or solvate thereof.

In other embodiments this invention encompasses the novel compounds of Formula I and the salts and solvates of those compounds, as well as pharmaceutical formulations comprising at least one compound of Formula I, or a pharmaceutically acceptable salt or solvent of said compound, in combination with one or more pharmaceutically acceptable carrier, diluents or excipients.

The terms and abbreviations used in the instant examples have their normal meanings unless otherwise designated. For example "°C" refers to degrees Celsius; "N" refers to normal or normality; "mmol" refers to millimole or millimoles; "g" refers to gram or grams; "ml" means milliliter or milliliters; "M" refers to molar or molarity; "MS" refers to mass spectrometry; "IR" refers to infrared spectroscopy; and "NMR" refers to nuclear magnetic resonance spectroscopy.

As used herein, the term "$C_1$-$C_6$ alkyl" refers to straight or branched, monovalent, saturated aliphatic chains of 1 to 6 carbon atoms and includes, but is not limited to, methyl, ethyl, propyl, isopropyl, butyl, isobutyl, t-butyl, pentyl, isopentyl, and hexyl. The term "$C_1$-$C_6$ alkyl" includes within its definition the term "$C_1$-$C_3$ alkyl".

"$C_1$-$C_6$ alkylidenyl" refers to a straight or branched, divalent, saturated aliphatic chains of 1 to 6 carbon atoms and includes, but is not limited to, methylenyl, ethylenyl, propylenyl, isopropylenyl, butylenyl, isobutylenyl, t-butylenyl, pentylenyl, isopentylenyl, and hexylenyl.

"Halo" represents chloro, fluoro, bromo or iodo.

"$C_1$-$C_6$ alkylthio" represents a straight or branched alkyl chain having from one to six carbon atoms attached to a sulfur atom. Typical $C_1$-$C_6$ alkylthio groups include methylthio, ethylthio, propylthio, isopropylthio, butylthio and the like. The term "$C_1$-$C_6$ alkylthio" includes within its definition the term "$C_1$-$C_3$ alkylthio".

The term "$C_2$-$C_8$ alkenyl" as used herein represents a straight or branched, monovalent, unsaturated aliphatic chain having from two to eight carbon atoms. Typical $C_2$-$C_6$ alkenyl groups include ethenyl (also known as vinyl), 1-methylethenyl, 1-methyl-1-propenyl, 1-butenyl, 1-hexenyl, 2-methyl-2-propenyl, 1-propenyl, 2-propenyl, 2-butenyl, 2-pentenyl, and the like.

"$C_5$-$C_8$ cycloalkenyl" represents a hydrocarbon ring structure containing from five to eight carbon atoms and having at least one double bond within that ring, which is unsubstituted or substituted with 1, 2 or 3 substituents independently selected from halo, halo($C_1$-$C_4$)alkyl, $C_1$-$C_4$ alkyl, $C_1$-$C_4$ alkoxy, carboxy, $C_1$-$C_4$ alkoxycarbonyl, carbamoyl, N-($C_1$-$C_4$)alkylcarbamoyl, amino, $C_1$-$C_4$ alkylamino, di($C_1$-$C_4$)alkylamino or $-(CH_2)_a-R^c$ where a is 1, 2, 3 or 4 and $R^c$ is hydroxy, $C_1$-$C_4$ alkoxy, carboxy, $C_1$-$C_4$ alkoxycarbonyl, amino, carbamoyl, $C_1$-$C_4$ alkylamino or di($C_1$-$C_4$)alkylamino.

"$C_1$-$C_6$ alkylamino" represents a straight or branched alkylamino chain having from one to six carbon atoms attached to an amino group. Typical $C_1$-$C_4$ alkyl-amino groups include methylamino, ethylamino, propylamino, isopropylamino, butylamino, sec-butylamino and the like. "$C_1$-$C_6$ alkylamino" encompasses within this term "$C_1$-$C_4$ alkylamino".

"Di($C_1$-$C_4$ alkyl)amino" represents a straight or branched dialkylamino chain having two alkyl chains, each having independently from one to four carbon atoms attached to a common amino group. Typical di($C_1$-$C_4$)alkylamino groups include dimethylamino, ethylmethylamino, methylisopropylamino, t-butylisopropylamino, di-t-butylamino and the like.

"$C_1$-$C_6$ alkoxy" represents a straight or branched alkyl chain having from one to six carbon atoms attached to an oxygen atom. Typical $C_1$-$C_6$ alkoxy groups include methoxy, ethoxy, propoxy, isopropoxy, butoxy, t-butoxy, pentoxy and the like. The term "$C_1$-$C_6$ alkoxy" includes within its definition the term "$C_1$-$C_3$ alkoxy".

"$C_2$-$C_6$ alkanoyl" represents a straight or branched alkyl chain having from one to five carbon atoms attached to a carbonyl moiety. Typical $C_2$-$C_6$ alkanoyl groups include ethanoyl, propanoyl, isopropanoyl, butanoyl, *t*-butanoyl, pentanoyl, hexanoyl, 3-methylpentanoyl and the like.

"$C_1$-$C_4$ alkoxycarbonyl" represents a straight or branched alkoxy chain having from one to four carbon atoms attached to a carbonyl moiety. Typical $C_1$-$C_4$ alkoxycarbonyl groups include methoxycarbonyl, ethoxycarbonyl, propoxycarbonyl, isopropoxycarbonyl, butoxycarbonyl, *t*-butoxycarbonyl and the like.

"$C_3$-$C_8$ cycloalkyl" represents a saturated hydrocarbon ring structure containing from three to eight carbon atoms. Typical $C_3$-$C_8$ cycloalkyl groups include cyclopropyl, cyclopentyl, cyclohexyl, cycloheptyl, and the like.

The term "amino-protecting group" as used in the specification refers to substituents of the amino group commonly employed to block or protect the amino functionality while reacting other functional groups on the compound. Examples of such amino-protecting groups include formyl, trityl, phthalimido, trichloroacetyl, chloroacetyl, bromoacetyl, iodoacetyl, and urethane-type blocking groups such as benzyloxycarbonyl, 4-phenylbenzyloxycarbonyl, 2-methylbenzyloxycarbonyl, 4-methoxybenzyloxycarbonyl, 4-fluorobenzyloxycarbonyl, 4-chlorobenzyloxycarbonyl, 3-chlorobenzyloxycarbonyl, 2-chlorobenzyloxycarbonyl, 2,4-dichlorobenzyloxycarbonyl, 4-bromobenzyloxycarbonyl, 3-bromobenzyloxycarbonyl, 4-nitrobenzyloxycarbonyl, 4-cyanobenzyloxycarbonyl, *t*-butoxycarbonyl, 1,1-diphenyleth-1-yloxycarbonyl, 1,1-diphenylprop-1-yloxycarbonyl, 2-phenylprop-2-yloxycarbonyl, 2-(*p*-toluyl)-prop-2-yloxycarbonyl, cyclopentanyloxycarbonyl, 1-methylcyclopentanyloxycarbonyl, cyclohexanyloxycarbonyl, 1-methylcyclohexanyloxycarbonyl, 2-methylecyclohexanyloxycarbonyl, 2-(4-toluylsulfonyl)-ethoxycarbonyl, 2-(methylsulfonyl)ethoxycarbonyl, 2-(triphenylphosphino)-ethoxycarbonyl, fluorenylmethoxy-carbonyl ("FMOC"), 2-(trimethylsilyl)ethoxycarbonyl, allyloxycarbonyl, 1-(trimethylsilylmethyl)prop-l-enyloxycarbonyl, 5-benzisoxalylmethoxycarbonyl, 4-acetoxybenzyloxycarbonyl, 2,2,2-trichloroethoxycarbonyl, 2-ethynyl-2-propoxycarbonyl, cyclopropylmethoxycarbonyl, 4-(decyloxy)benzyloxycarbonyl, isobornyloxycarbonyl, 1-piperidyloxycarbonyl and the like; benzoylmethylsulfonyl group, 2-nitrophenylsulfenyl, diphenylphosphine oxide and like amino-protecting groups. The species of amino-protecting group employed is usually not critical so long as the derivatized amino group is stable to the condition of subsequent reactions on other positions of the intermediate molecule and can be selectively removed at the appropriate point without disrupting the remainder of the molecule including any other amino-protecting groups. Preferred amino-protecting groups are trityl, *t*-butoxycarbonyl (*t*-BOC), allyloxycarbonyl and benzyloxycarbonyl. Further examples of groups referred to by the above terms are described by E. Haslam, "Protective Groups in Organic Chemistry", (J.G.W. McOmie, ed., 1973), at Chapter 2; and T.W. Greene and P.G.M. Wuts, "Protective Groups

in Organic Synthesis" (1991), at Chapter 7.

The term "carboxy-protecting group" as used in the specification refers to substituents of the carboxy group commonly employed to block or protect the carboxy functionality while reacting other functional groups on the compound. Examples of such carboxy-protecting groups include methyl, *p*-nitrobenzyl, *p*-methylbenzyl, *p*-methoxy-benzyl, 3,4-dimethoxybenzyl, 2,4-dimethoxybenzyl, 2,4,6-trimethoxybenzyl, 2,4,6-trimethylbenzyl, pentamethylbenzyl, 3,4-methylene-dioxybenzyl, benzhydryl, 4,4'-dimethoxy-benzhydryl, 2,2',4,4'-tetramethoxybenzhydryl, *t*-butyl, *t*-amyl, trityl, 4-methoxytrityl, 4,4'-dimethoxytrityl, 4,4',4"-trimethoxytrityl, 2-phenylprop-2-yl, trimethylsilyl, *t*-butyldimethylsilyl, phenacyl, 2,2,2-trichloroethyl, 2-(di(n-butyl)methylsilyl)ethyl, *p*-toluenesulfonylethyl, 4-nitrobenzylsulfonylethyl, allyl, cinnamyl, 1-(trimethylsilylmethyl)prop-1-en-3-yl and like moieties. Preferred carboxy-protecting groups are allyl, benzyl and *t*-butyl. Further examples of these groups are found in E. Haslam, supra, at Chapter 5, and T.W. Greene, et al., supra, at Chapter 5.

The term "leaving group" as used herein refers to a group of atoms that is displaced from a carbon atom by the attack of a nucleophile in a nucleophilic substitution reaction. The term "leaving group" as used in this document encompasses, but is not limited to, activating groups.

The term "activating group" as used herein refers a leaving group which, when taken with the carbonyl (-C=O) group to which it is attached, is more likely to take part in an acylation reaction than would be the case if the group were not present, as in the free acid. Such activating groups are well-known to those skilled in the art and may be, for example, succinimidoxy, phthalimidoxy, benzotriazolyloxy, benzenesulfonyloxy, methanesulfonyloxy, toluenesulfonyloxy, azido, or -O-CO-($C_4$-$C_7$ alkyl).

The term "haloformate" as used herein refers to an ester of a haloformic acid, this compound having the formula

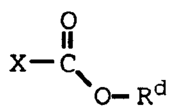

wherein X is halo, and $R^d$ is $C_1$-$C_6$ alkyl. Preferred haloformates are bromoformates and chloroformates. Especially preferred are chloroformates. Those haloformates wherein $R^d$ is $C_3$-$C_6$ are preferred. Most preferred is isobutylchloroformate.

The compounds used in the method of the present invention have multiple asymmetric centers. As a consequence of these chiral centers, the compounds of the present invention occur as racemates, mixtures of enantiomers and as individual enantiomers, as well as diastereomers and mixtures of diastereomers. All asymmetric forms, individual isomers and combinations thereof, are within the scope of the present invention.

The terms "R" and "S" are used herein as commonly used in organic chemistry to denote specific configuration of a chiral center. The term "R" *(rectus)* refers to that configuration of a chiral center with a clockwise relationship of group priorities (highest to second lowest) when viewed along the bond toward the lowest priority group. The term "S" *(sinister)* refers to that configuration of a chiral center with a counterclockwise relationship of group priorities (highest to second lowest) when viewed along the bond toward the lowest priority group. The priority of groups is based upon their atomic number (in order of decreasing atomic number). A partial list of priorities and a discussion of stereochemistry is contained in "Nomenclature of Organic Compounds: Principles and Practice", (J.H. Fletcher, et al., eds., 1974) at pages 103-120.

In addition to the (R)-(S) system, the older D-L system is also used in this document to denote absolute configuration, especially with reference to amino acids. In this system a Fischer projection formula is oriented so that the number 1 carbon of the main chain is at the top. The prefix "D" is used to represent the absolute configuration of the isomer in which the functional (determining) group is on the right side of the carbon atom at the chiral center and "L", that of the isomer in which it is on the left.

In addition to the (R)-(S) steroisomerism, the presence of at least two substituents on the ring of the cycloalkyl molecule allows for the possibility of cis-trans isomerism. Especially preferred in this invention are those compounds of Formula I wherein the substituted amino group at position 2 of the cyclohexyl ring is in a trans relationship to the substituted thio, amino, or oxy group at position 1 as depicted below.

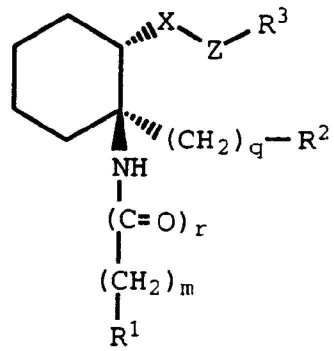

As noted supra, all asymmetric forms, individual isomers and combinations thereof, are within the scope of the present invention.

In order to preferentially prepare one optical isomer over its enantiomer, the skilled practitioner can proceed by one of two routes. The practitioner may first prepare the mixture of enantiomers and then separate the two enantiomers. A commonly employed method for the resolution of the racemic mixture (or mixture of enantiomers) into the individual enantiomers is to first convert the enantiomers to diastereomers by way of forming a salt with an optically active salt or base. These diastereomers can then be separated using differential solubility, fractional crystallization, chromatography, or like methods. Further details regarding resolution of enantiomeric mixtures can be found in J. Jacques, et al., "Enantiomers, Racemates, and Resolutions", (1991).

In addition to the schemes described above, the practitioner of this invention may also choose an enantiospecific protocol for the preparation of the compounds of Formula I. Such a protocol employs a synthetic reaction design which maintains the chiral center present in the starting material in a desired orientation. These reaction schemes usually produce compounds in which greater than 95 percent of the title product is the desired enantiomer.

As noted supra, this invention includes the pharmaceutically acceptable salts of the compounds defined by Formula I. A compound of this invention can possess a sufficiently acidic, a sufficiently basic, or both functional groups, and accordingly react with any of a number of organic and inorganic bases, and inorganic and organic acids, to form a pharmaceutically acceptable salt.

The term "pharmaceutically acceptable salt" as used herein, refers to salts of the compounds of the above formula which are substantially non-toxic to living organisms. Typical pharmaceutically acceptable salts include those salts prepared by reaction of the compounds of the present invention with a pharmaceutically acceptable mineral or organic acid or an organic or inorganic base. Such salts are known as acid addition and base addition salts.

Acids commonly employed to form acid addition salts are inorganic acids such as hydrochloric acid, hydrobromic acid, hydroiodic acid, sulfuric acid, phosphoric acid, and the like, and organic acids such as *p*-toluenesulfonic acid, methanesulfonic acid, oxalic acid, *p*-bromophenylsulfonic acid, carbonic acid, succinic acid, citric acid, benzoic acid, acetic acid, and the like. Examples of such pharmaceutically acceptable salts are the sulfate, pyrosulfate, bisulfate, sulfite, bisulfite, phosphate, monohydrogenphosphate, dihydrogenphosphate, metaphosphate, pyrophosphate, bromide, iodide, acetate, propionate, decanoate, caprylate, acrylate, formate, hydrochloride, dihydrochloride, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, hydroxybenzoate, methoxybenzoate, phthalate, xylenesulfonate, phenylacetate, phenylpropionate, phenylbutyrate, citrate, lactate, γ-hydroxybutyrate, glycolate, tartrate, methanesulfonate, propanesulfonate, naphthalene-l-sulfonate, napththalene-2-sulfonate, mandelate and the like. Preferred pharmaceutically acceptable acid addition salts are those formed with mineral acids such as hydrochloric acid and hydrobromic acid, and those formed with organic acids such as maleic acid and methanesulfonic acid.

Salts of amine groups may also comprise quarternary ammonium salts in which the amino nitrogen carries a suitable organic group such as an alkyl, alkenyl, alkynyl, or aralkyl moiety.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred.

It should be recognized that the particular counterion forming a part of any salt of this invention is usually

not of a critical nature, so long as the salt as a whole is pharmacologically acceptable and as long as the counterion does not contribute undesired qualities to the salt as a whole.

This invention further encompasses the pharmaceutically acceptable solvates of the compounds of Formulas I. Many of the Formula I compounds can combine with solvents such as water, methanol, ethanol and acetonitrile to form pharmaceutically acceptable solvates such as the corresponding hydrate, methanolate, ethanolate and acetonitrilate.

This invention also encompasses the pharmaceutically acceptable prodrugs of the compounds of Formula I. A prodrug is a drug which has been chemically modified and may be biologically inactive at its site of action, but which may be degraded or modified by one or more enzymatic or other in vivo processes to the parent bioactive form. This prodrug should have a different pharmacokinetic profile than the parent, enabling easier absorption across the mucosal epithelium, better salt formation or solubility, or improved systemic stability (an increase in plasma half-life, for example).

Typically, such chemical modifications include:

1) ester or amide derivatives which may be cleaved by esterases or lipases;

2) peptides which may be recognized by specific or nonspecific proteases; or

3) derivatives that accumulate at a site of action through membrane selection of a prodrug form or a modified prodrug form;

or any combination of 1 to 3, supra. Conventional procedures for the selection and preparation of suitable prodrug derivatives are described, for example, in H, Bundgaard, Design of Prodrugs, (1985).

The especially preferred methods of this invention are those methods employing compounds wherein:

a) $R^2$ is phenyl, 3-indolyl, optionally substituted with one or two substituents independently selected from the group consisting of methyl, ethyl, methoxy, ethoxy, chloro, fluoro and trifluoromethyl;

b) Z is $-CHR^4-$;

c) $R^1$ is hydrogen, phenyl, substituted phenyl, piperidinyl, substituted piperidinyl, piperazinyl, substituted piperazinyl, pyrrolidinyl, substituted pyrrolidinyl, pyridyl, benzoyl, or morpholinyl;

d) $R^3$ is phenyl, substituted phenyl, $C_3$-$C_8$ cycloalkyl, substituted $C_3$-$C_8$ cycloalkyl, naphthyl or substituted naphthyl;

e) X is -O-, or -NH-;

f) r is 1; and

g) $R^4$ is hydrogen.

A most preferred group of compounds used in the methods of this invention are those of Formula I wherein $R^2$ is unsubstituted phenyl or 3-indolyl, $R^1$ is substituted piperidinyl or substituted piperazinyl, and $R^3$ is mono- or disubstituted phenyl.

The compounds of the present invention can be prepared by a variety of procedures well known to those of ordinary skill in the art. The particular order of steps required to produce the compounds of Formula I is dependent upon the particular compound being synthesized, the starting compound, and the relative lability of the substituted moieties.

The term "optionally in the presence of a base" indicates that the reaction may be performed with a base present, but such a base is not required for the reaction to proceed. Preferred bases include organic bases containing one or more nitrogen groups, such as N-methylmorpholine, ethylamine, diethylamine, triethylamine, ethyldiisopropylamine, pyridine, and the like. Especially preferred are N-methylmorpholine and pyridine. The absence of a base is usually most preferred.

A preferred process for preparing the compounds of Formula I where r=1 is by the acylation of the primary amine of a compound of Formula II

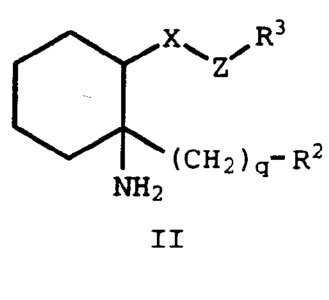

II

or a salt or solvate thereof.

The acylation of this primary amine can be accomplished by a number of methods known in the art. In those compounds of Formula II in which X is -NH- and $Z$-$R^3$ is not -H, the primary amine can be selectively

acylated by various means. This acylation can be done using any of a large number of techniques regularly employed by those skilled in organic chemistry. One such reaction scheme is a substitution using an anhydride such as acetic anhydride. Another reaction scheme often employed to acylate a primary amine employs a carboxylic acid of Formula III

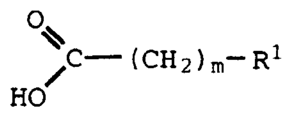

III

or a salt or solvate thereof, preferably with an activating agent, such as 1,1-carbonyl diimidazole, dicyclohexylcarbodiimide, diethyl azodicarboxylate, 1-hydroxybenzotriazole, alkyl chloroformate and triethylamine, phenyldichlorophosphate, and chlorosulfonyl isocyanate.

An amino-de-alkoxylation type of reaction uses esters as a means of acylating the primary amine. Activated esters which are attenuated to provide enhanced selectivity are very efficient acylating agents.

In an especially preferred embodiment, a compound of Formula III, or a salt thereof, is first reacted with a suitable haloformate and this is then reacted with a compound of Formula II, or a salt thereof, optionally in the presence of a base.

A preferred process for preparing the compounds of Formula II is described in PCT Publication WO 94/07843, published April 14, 1994. In this process a compound of Formula IV

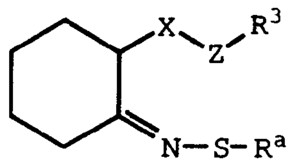

IV

where $R^a$ is $C_1$-$C_6$ alkyl or, preferably, a phenyl group, is reacted with a reagent of formula $R^2$-$(CH_2)_q$-M where M represents an alkali metal, such as lithium. This reaction is conveniently effected in a suitable organic solvent, such as an ether, for example, tetrahydrofuran.

The intermediates of Formula IV may be prepared from compounds of Formula V

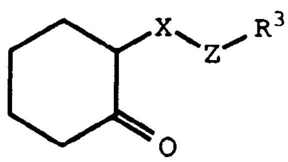

V

by reaction with a compound of Formula VI

$R^a$-S-S-$R^a$ VI

in the presence of ammonia and a nitrite, such as, for example, silver nitrite.

Compounds of Formula V may be prepared by the oxidation of the corresponding alcohols of Formula VII

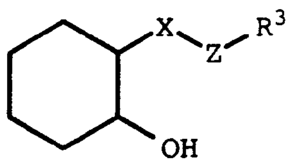

VII

by conventional methods. Usually the oxidation is effected under Swern condition, i.e. with the use of oxalyl chloride in the presence of dimethyl sulfoxide. Other suitable oxidation procedures will readily apparent to those skilled in the art.

A compound of Formula VII may be prepared by the reaction of a compound of Formula VIII

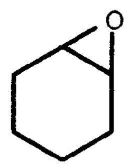

VIII

with a compound of Formula IX

$HX\text{-}Z\text{-}R^3$ IX

in the presence of a base or catalyst. Suitable agents for use in this reaction include metal hydrides, such as, for example, potassium hydride, or alumina.

During any of the above synthetic sequences it may be necessary or desirable to protect sensitive or reactive amino or carboxy groups on any of the molecules concerned. Such protection may be accomplished by means of the amino-protecting or carboxy-protecting groups discussed supra. These protecting groups may be removed at a convenient subsequent stage using methods known in the art.

Preparation 1

Preparation of 1-(3,5-dimethylbenzyloxy)-2-amino-2-phenylcyclohexane.

A toluene solution (70 ml) containing cyclohexane oxide (20 g), 3,5-dimethylbenzyl alcohol and alumina is heated at reflux for about 16 hours with azeotrophic removal of water. The solution is filtered and the solvent removed in vacuo to give 1-(3,5-dimethylbenzyloxy)-2-hydroxycyclohexane as as oil.

The 1-(3,5-dimethylbenzyloxy)-2-hydroxycyclohexane is oxidized under standard Swern conditions [as described in Journal of Organic Chemistry, 43:2480 (1978)] using oxalyl chloride (4.12 ml) and dimethyl sulfoxide (6.7 ml). The product is purified on silica gel eluting with petroleum ether/ethyl acetate mixtures to give 1-(3,5-dimethylbenzyloxy)-2-cyclohexanone as an oil.

The 1-(3,5-dimethylbenzyloxy)-2-cyclohexanone (10 g) is converted into the corresponding sulfenimine using the procedure described in Davis, Journal of Organic Chemistry, 38:2809 (1973) by treatment with silver nitrate (4.9 g), phenyl disulfide (6.5 g) and ammonia. The crude product is purified on silica gel eluting with petroleum ether/ethyl acetate mixtures to give 1-(3,5-dimethylbenzyloxy)-2-phenyl sulfenimine cyclohexane.

The 1-(3,5-dimethylbenzyloxy)-2-phenyl sulfenimine cyclohexane (5.80 g) is then dissolved in ether (100 ml) at 0°C. Phenyllithium (17.1 ml) is added and after one hour the reaction mixture is heated to reflux. The reaction is quenched with 2M sodium hydroxide (100 ml) and the product is extracted into ethyl acetate (3 x 50 ml). The combined organic phase is washed with water (2 x 50 ml), saturated sodium chloride (50 ml), dried over magnesium sulfate, and evaporated in vacuo. The product is further purified on silica eluting with petroleum ether/ethyl acetate mixtures to give 1-(3,4-dimethylbenzyloxy)-2-amino-2-phenylcyclohexane as a crystalline solid.

Preparation 2

Preparation of 1-(3,5-ditrifluoromethylbenzyloxy)-2-amino-2-phenylcyclohexane

The title intermediate is prepared essentially as described in Preparation 1 but using 3,5-ditrifluoromethylbenzyl alcohol in place of 3,5-dimethylbenzyl alcohol.

Preparation 3

Preparation of 1-(3,5-dimethoxybenzyloxy)-2-amino-2-phenylcyclohexane

The title intermediate is prepared essentially as described in Preparation 1 but using 3,5-dimethoxybenzyl alcohol in place of 3,5-dimethylbenzyl alcohol.

Preparation 4

Preparation of 1-(3,5-dichlorobenzyloxy)-2-amino-2-phenylcyclohexane

The title intermediate is prepared essentially as described in Preparation 1 but using 3,5-dichlorobenzyl alcohol in place of 3,5-dimethylbenzyl alcohol.

Preparation 5

Preparation of 1-(2-methoxybenzyloxy)-2-amino-2-phenylcyclohexane

The title intermediate is prepared essentially as described in Preparation 1 but using 2-methoxybenzyl alcohol in place of 3,5-dimethylbenzyl alcohol.

Preparation 6

Preparation of 1-(2-chlorobenzyloxy)-2-amino-2-phenylcyclohexane

The title intermediate is prepared essentially as described in Preparation 1 but using 2-chlorobenzyl alcohol in place of 3,5-dimethylbenzyl alcohol.

Preparation 7

Preparation of 1-(2-methylbenzyloxy)-2-amino-2-phenylcyclohexane

The title intermediate is prepared essentially as described in Preparation 1 but using 2-methylbenzyl alcohol in place of 3,5-dimethylbenzyl alcohol.

Preparation 8

Preparation of 1-(2-trifluoromethylbenzyloxy)-2-amino-2-phenylcyclohexane

The title intermediate is prepared essentially as described in Preparation 1 but using 2-trifluoromethylbenzyl alcohol in place of 3,5-dimethylbenzyl alcohol.

Preparation 9

Preparation of 1-(3,5-dimethylbenzylamino)-2-amino-2-phenylcyclohexane

The title intermediate is prepared essentially as described in Preparation 1 but using 3,5-dimethylbenzylamine in place of 3,5-dimethylbenzyl alcohol. Journal of the American Chemical Society, 99:8208 (1977); Synthesis, 1984:629; Tetrahedron Letters, 1975:3577.

Preparation 10

Preparation of 1-(3,5-ditrifluoromethylbenzylamino)-2-amino-2-phenylcyclohexane

The title intermediate is prepared essentially as described in Preparation 2 but using 3,5-ditrifluoromethylbenzylamine in place of 3,5-ditrifluoromethylbenzyl alcohol.

Preparation 11

Preparation of 1-(3,5-dimethoxybenzylamino)-2-amino-2-phenylcyclohexane

The title intermediate is prepared essentially as described in Preparation 3 but using 3,5-dimethoxybenzylamine in place of 3,5-dimethoxybenzyl alcohol.

Preparation 12

Preparation of 1-(3,5-dichlorobenzylamino)-2-amino-2-phenylcyclohexane

The title intermediate is prepared essentially as described in Preparation 4 but using 3,5-dichlorobenzylamine in place of 3,5-dichlorobenzyl alcohol.

Preparation 13

Preparation of 1-(2-methoxybenzylamino)-2-amino-2-phenylcyclohexane

The title intermediate is prepared essentially as described in Preparation 5 but using 2-methoxybenzylamine in place of 2-methoxybenzyl alcohol.

Preparation 14

Preparation of 1-(2-chlorobenzylamino)-2-amino-2-phenylcyclohexane

The title intermediate is prepared essentially as described in Preparation 1 but using 2-chlorobenzylamine in place of 2-chlorobenzyl alcohol.

Preparation 15

Preparation of 1-(2-methylbenzylamino)-2-amino-2-phenylcyclohexane

The title intermediate is prepared essentially as described in Preparation 7 but using 2-methylbenzylamine in place of 2-methylbenzyl alcohol.

Preparation 16

Preparation of 1-(2-trifluoromethylbenzylamino)-2-amino-2-phenylcyclohexane

The title intermediate is prepared essentially as described in Preparation 8 but using 2-trifluoromethylbenzylamine in place of 2-trifluoromethylbenzyl alcohol.

Preparation 17

Preparation of 1-(3,5-dimethylbenzyloxy)-2-amino-2-(3-(1H-indolyl))cyclohexane

The title intermediate is prepared essentially as described in Preparation 1 but 1H-indole, magnesium bromide salt is employed in place of the phenyllithium.

Preparation 18

Preparation of 1-(3,5-ditrifluoromethylbenzyloxy)-2-amino-2-(3-(1H-indolyl))cyclohexane

The title intermediate is prepared essentially as described in Preparation 2 but 1H-indole, magnesium bromide salt is employed in place of the phenyllithium.

Preparation 19

Preparation of 1-(3,5-dimethoxybenzyloxy)-2-amino-2-(3-(1H-indolyl))cyclohexane

The title intermediate is prepared essentially as described in Preparation 3 but 1H-indole, magnesium bromide salt is employed in place of the phenyllithium.

Preparation 20

Preparation of 1-(3,5-dichlorobenzyloxy)-2-amino-2-(3-(1H-indolyl))cyclohexane

The title intermediate is prepared essentially as described in Preparation 4 but 1H-indole, magnesium bromide salt is employed in place of the phenyllithium.

Preparation 21

Preparation of 1-(2-methoxybenzyloxy)-2-amino-2-(3-(1H-indolyl))cyclohexane

The title intermediate is prepared essentially as described in Preparation 5 but 1H-indole, magnesium bromide salt is employed in place of the phenyllithium.

Preparation 22

Preparation of 1-(2-chlorobenzyloxy)-2-amino-2-(3-(1H-indolyl))cyclohexane

The title intermediate is prepared essentially as described in Preparation 6 but 1H-indole, magnesium bromide salt is employed in place of the phenyllithium.

Preparation 23

Preparation of 1-(2-methylbenzyloxy)-2-amino-2-(3-(1H-indolyl))cyclohexane

The title intermediate is prepared essentially as described in Preparation 7 but 1H-indole, magnesium bromide salt is employed in place of the phenyllithium.

Preparation 24

Preparation of 1-(2-trifluoromethylbenzyloxy)-2-amino-2-(3-(1H-indolyl))cyclohexane

The title intermediate is prepared essentially as described in Preparation 8 but 1H-indole, magnesium bromide salt is employed in place of the phenyllithium.

Preparation 25

Preparation of 1-(3,5-dimethylbenzylamino)-2-amino-2-(3-(1H-indolyl))cyclohexane

The title intermediate is prepared essentially as described in Preparation 9 but 1H-indole, magnesium bromide salt is employed in place of the phenyllithium.

Preparation 26

Preparation of 1(3,5-ditrifluoromethylbenzylamino)-2-amino-2-(3-(1H-indolyl))cyclohexane

The title intermediate is prepared essentially as described in Preparation 10 but 1H-indole, magnesium bromide salt is employed in place of the phenyllithium.

Preparation 27

Preparation of 1-(3,5-dimethoxybenzylamino)-2-amino-2-(3-(1H-indolyl))cyclohexane

The title intermediate is prepared essentially as described in Preparation 11 but 1H-indole, magnesium bromide salt is employed in place of the phenyllithium.

Preparation 28

Preparation of 1-(3,5-dichlorobenzylamino)-2-amino-2-(3-(1H-indolyl))cyclohexane

The title intermediate is prepared essentially as described in Preparation 12 but 1H-indole, magnesium bromide salt is employed in place of the phenyllithium.

Preparation 29

Preparation of 1-(2-methoxybenzylamino)-2-amino-2-(3-(1H-indolyl))cyclohexane

The title intermediate is prepared essentially as described in Preparation 13 but 1H-indole, magnesium bromide salt is employed in place of the phenyllithium.

Preparation 30

Preparation of 1-(2-chlorobenzylamine)-2-amino-2-(3-(1H-indolyl))cyclohexane

The title intermediate is prepared essentially as described in Preparation 14 but 1H-indole, magnesium bromide salt is employed in place of the phenyllithium.

Preparation 31

Preparation of 1-(2-methylbenzylamino)-2-amino-2-(3-(1H-indolyl))cyclohexane

The title intermediate is prepared essentially as described in Preparation 15 but 1H-indole, magnesium bromide salt is employed in place of the phenyllithium.

Preparation 32

Preparation of 1-(2-trifluoromethylbenzylamino)-2-amino-2-(3-(1H-indolyl))cyclohexane

The title intermediate is prepared essentially as described in Preparation 16 but 1H-indole, magnesium bromide salt is employed in place of the phenyllithium.

Preparation 33

Preparation of 1-(3,5-dimethylbenzylthio)-2-amino-2-phenylcyclohexane

The title intermediate is prepared essentially as described in Preparation 1 but using 3,5-ditrifluoromethylbenzyl mercapatan in place of 3,5-dimethylbenzyl alcohol.

Preparation 34

Preparation of 1-(3,5-ditrifluoromethylbenzylthio)-2-amino-2-phenylcyclohexane

The title intermediate is prepared essentially as described in Preparation 33 but using 3,5-ditrifluoromethylbenzyl mercapatan in place of 3,5-dimethylbenzyl mercapatan.

Preparation 35

Preparation of 1-(3,5-dimethoxybenzylthio)-2-amino-2-phenylcyclohexane

The title intermediate is prepared essentially as described in Preparation 33 but using 3,5-dimethoxybenzyl mercapatan in place of 3,5-dimethylbenzyl mercapatan.

Preparation 36

Preparation of 1-(3,5-dichlorobenzylthio)-2-amino-2-phenylcyclohexane

The title intermediate is prepared essentially as described in Preparation 33 but using 3,5-dichlorobenzyl mercapatan in place of 3,5-dimethylbenzyl mercapatan.

Preparation 37

Preparation of 1-(2-methoxybenzylthio)-2-amino-2-phenylcyclohexane

The title intermediate is prepared essentially as described in Preparation 33 but using 2-methoxybenzyl mercapatan in place of 3,5-dimethylbenzyl mercapatan.

Preparation 38

Preparation of 1-(2-chlorobenzylthio)-2-amino-2-phenylcyclohexane

The title intermediate is prepared essentially as described in Preparation 33 but using 2-chlorobenzyl mercapatan in place of 3,5-dimethylbenzyl mercapatan.

Preparation 39

Preparation of 1-(2-methylbenzylthio)-2-amino-2-phenylcyclohexane

The title intermediate is prepared essentially as described in Preparation 33 but using 2-methylbenzyl mercapatan in place of 3,5-dimethylbenzyl mercapatan.

Preparation 40

Preparation of 1-(2-trifluoromethylbenzylthio)-2-amino-2-phenylcyclohexane

The title intermediate is prepared essentially as described in Preparation 33 but using 2-trifluoromethylbenzyl mercapatan in place of 3,5-dimethylbenzyl mercapatan.

In all of the above examples the compounds containing a 3,4-disubstituted benzyl- thio, amino, or oxy or a 2,4-disubstitutedbenzyl- thio, amino, or oxy may be prepared essentially as described above by employing the appropriately substituted benzyl mercaptan, benzylamine, or benzyl alcohol.

Preparation 41

Preparation of 2-((4-cyclohexyl)piperazin-1-yl)acetic acid potassium salt hydrate

Cyclohexylpiperazine (10.0 g, 0.059 mol) is added to ten volumes of methylene chloride at room temperature. To this mixture is added sodium hydroxide (36 ml of a 2N solution, 0.072 mol) and tetrabutylammonium

bromide (1.3 g, 0.004 mol). After the addition of the sodium hydroxide and tetrabutylammonium bromide, methyl bromoacetate (7.0 ml, 0.073 mol) is added and the reaction mixture is stirred for four to six hours. The progress of the reaction is monitored by gas chromatography.

The organic fraction is separated and the aqueous phase is back-extracted with methylene chloride. The organic phases are combined and washed twice with deionized water, once with saturated sodium bicarbonate solution, and then with brine. The organic phase is dried over magnesium sulfate and the solvents are removed in vacuo to yield 2-((4-cyclohexyl)piperazin-1-yl)methyl acetate as a yellowish oil.

The title intermediate is prepared by dissolving the 2-((4-cyclohexyl)piperazin-1-yl)methyl acetate (10.0 g, 0.042 mol) in ten volumes of diethyl ether. This solution is cooled to 15°C and then potassium trimethylsilanoate (5.9 g, 0.044) is added. This mixture is then stirred for four to six hours. The reaction product is removed by filtration, washed twice with five volumes of diethyl ether, then washed twice with five volumes of hexanes, and then dried in a vacuum oven for 12-24 hours at 50°C.

Preparation 42

Preparation of 2-(4-(piperidin-1-yl)piperidin-1-yl)acetic acid, potassium salt

4-Piperidinopiperidine (1.20 kg, 7.13 mol) is added to methylene chloride (12.0 L) under a nitrogen atmosphere. Tetrabutylammonium bromide (0.150 kg, 0.47 mol) and sodium hydroxide (1.7 L of a 5 N solution, 8.5 mol) are then added. The reaction mixture is cooled to 10-15°C and methyl bromoacetate (1.17 kg, 7.65 mol) is added and the resulting mixture is stirred for a minimum of 16 hours.

Deionized water (1.2 L) is then added to the mixture and the layers separated. The aqueous layer is back-extracted with methylene chloride (2.4 L). The organic fractions are combined and washed with deionized water (3 x 1.2 L), a saturated sodium bicarbonate solution (1.1 L) and a saturated sodium chloride solution (1.1 L). The organic fraction is then dried over anhydrous magnesium sulfate and concentrated to an oil on a rotary evaporator to yield 2-(4-(piperidin-1-yl)piperidin-1-yl)methyl acetate.

A solution of 2-(4-(piperidin-1-yl)piperidin-1-yl)methyl acetate (2.395 kg, 9.96 mol) in methanol (2.4 L) is added to a solution of potassium hydroxide (0.662 kg, 10.0 mol @ 85% purity) in methanol (10.5 L) under a nitrogen atmosphere. The reaction mixture is heated to 45-50°C for a minimum of 16 hours.

A solvent exchange from methanol to acetone (15.0 L) is performed on the solution on a rotary evaporator. This solution is slowly cooled to room temperature over 16 hours. The resulting solids are filtered, rinsed with acetone (5.0 L) and then dried to yield 2.471 kg (92%) of 2-(4-(piperidin-1-yl)piperidin-1-yl)acetic acid, potassium salt.

Example 1

Preparation of 1-(3,5-ditrifluoromethylbenzyloxy)-2-[2-[(4-cyclohexyl)piperazin-1-yl]acetylamino-2-phenylcyclohexane

The title compound is prepared by first cooling 2-((4-cyclohexyl)piperazin-1-yl)acetic acid potassium salt to a temperature between -8°C and -15°C in 5 volumes of anhydrous methylene chloride. To this mixture is added isobutylchloroformate at a rate such that the temperature does not exceed -8°C. The resulting reaction mixture is stirred for about 1 hour, the temperature being maintained between -8°C and -15°C.

To this mixture is then added 1-(3,5-ditrifluoromethylbenzyloxy)-2-amino-2-phenylcyclohexane at such a rate that the temperature does not exceed 0°C. Next added to this mixture is N-methyl morpholine at a rate such that the temperature does not exceed 0°C. This mixture is then stirred for about 1 hour at a temperature between -15°C and -8°C.

The reaction is quenched by the addition of 5 volumes of water. The organic layer is washed once with a saturated sodium bicarbonate solution. The organic phase is then dried over anhydrous potassium carbonate and filtered to remove the drying agent. To the filtrate is then added 2 equivalents of concentrated hydrochloric acid, followed by 1 volume of isopropyl alcohol. The methylene chloride is then exchanged with isopropyl alcohol under vacuum by distillation.

The final volume of isopropyl alcohol is then concentrated to three volumes by vacuum. The reaction mixture is cooled to 20°C to 25°C and the product is allowed to crystallize for at least one hour. The desired product is then recovered by filtration and washed with sufficient isopropyl alcohol to give a colorless filtrate. The crystal cake is then dried under vacuum at 50°C.

Example 2

Preparation of 1-(3,5-ditrifluoromethylbenzyloxy)-2-[2-[(4-phenyl)piperazin-1-yl]acetylamino-2-phenylcyclohexane

The title compound is prepared essentially as described in Example 1, except that 2-((4-phenyl)piperazin-1-yl)acetic acid, sodium salt is employed instead of 2-((4-cyclohexyl)piperazin-1-yl)acetic acid potassium salt.

Example 3

Preparation of 1-(3,5-dimethylbenzylamino)-2-[2-[(4-cyclohexyl)piperazin-1-yl]acetylamino-2-(3-1H-indolyl)cyclohexane

The title compound is prepared essentially as described in Example 1, except that the intermediate 1-(3,5-dimethylbenzylamino)-2-amino-2-(3-1H-indolyl)cyclohexane as prepared in Preparation 25 is employed instead of 1-(3,5-ditrifluoromethylbenzyloxy)-2-amino-2-phenylcyclohexane.

Example 4

Preparation of 1-(3,5-ditrifluoromethylbenzylamino)-2-[2-[(4-cyclohexyl)piperazin-1-yl]acetylamino-2-(3-1H-indolyl)cyclohexane

The title compound is prepared essentially as described in Example 1, except that the intermediate 1-(3,5-dimethylbenzylamino)-2-amino-2-[3-(1H-indolyl)]cyclohexane as prepared in Preparation 26 is employed instead of 1-(3,5-ditrifluoromethylbenzyloxy)-2-amino-2-phenylcyclohexane.

Example 5

Preparation of 1-(3,5-dimethylbenzylamino)-2-[2-[(4-piperidin-1-yl)piperidin-1-yl]acetylamino-2-[3-(1H-indolyl)]cyclohexane

The title compound is prepared essentially as described in Example 3, except that 2-(4-(piperidin-1-yl)piperidin-1-yl)acetic acid, potassium salt, is employed instead of 2-((4-cyclohexyl)piperazin-1-yl)acetic acid potassium salt.

Example 6

Preparation of 1-(2-methoxybenzylamino)-2-[2-[(4-piperidin-1-yl)piperidin-l-yl]acetylamino-2-(3-1H-indolyl)cyclohexane

The title compound is prepared essentially as described in Example 5, except that the intermediate 1-(2-methoxybenzylamino)-2-amino-2-(3-(1H-indolyl))cyclohexane, as prepared in Preparation 29, is employed instead of 1-(3,5-dimethylbenzylamino)-2-amino-2-(3-(1H-indolyl))cyclohexane.

Example 7

Preparation of 1-(3,5-ditrifluoromethylbenzylamino)-2-[2-[(4-piperidin-1-yl)piperidin-1-yl]acetylamino-2-(3-1H-indolyl)cyclohexane

The title compound is prepared essentially as described in Example 5, except that the intermediate 1-(3,5-ditrifluoromethylbenzylamino)-2-amino-2-(3-(1H-indolyl))cyclohexane, as prepared in Preparation 26, is employed instead of 1-(3,5-dimethylbenzylamino)-2-amino-2-(3-(1H-indolyl))cyclohexane.

The biological activity of the compounds of the present invention is evaluated employing an initial screening assay which rapidly and accurately measured the binding of the tested compound to known NK-1 and NK-2 receptor sites. Assays useful for evaluating tachykinin receptor antagonists are well known in the art. See. e.g., J. Jukic, et al., Life Sciences, 49:1463-1469 (1991); N. Kucharczyk, et al., Journal of Medicinal Chemistry, 36:1654-1661 (1993); N. Rouissi, et al., Biochemical and Biophysical Research Communications, 176:894-901 (1991).

NK-1 Receptor Binding Assay

Radioreceptor binding assays are performed using a derivative of a previously published protocol. D.G. Payan, et al., Journal of Immunology, 133:3260-3265 (1984). In this assay an aliquot of IM9 cells (1 x $10^6$ cells/tube in RPMI 1604 medium supplemented with 10% fetal calf serum) is incubated with 20 pM $^{125}$I-labeled substance P in the presence of increasing competitor concentrations for 45 minutes at 4°C.

The IM9 cell line is a well-characterized cell line which is readily available to the public. See. e.g., Annals of the New York Academy of Science, 190: 221-234 (1972); Nature (London), 251:443-444 (1974); Proceedings of the National Academy of Sciences (USA), 71:84-88 (1974). These cells are routinely cultured in RPMI 1640 supplemented with 50 μg/ml gentamicin sulfate and 10% fetal calf serum.

The reaction is terminated by filtration through a glass fiber filter harvesting system using filters previously soaked for 20 minutes in 0.1% polyethylenimine. Specific binding of labeled substance P is determined in the presence of 20 nM unlabeled ligand.

Many of the compounds employed in the methods of the present invention are also effective antagonists of the NK-2 receptor.

NK-2 Receptor Binding Assay

The CHO-hNK-2R cells, a CHO-derived cell line transformed with the human NK-2 receptor, expressing about 400,000 such receptors per cell, are grown in 75 $cm^2$ flasks or roller bottles in minimal essential medium (alpha modification) with 10% fetal bovine serum. The gene sequence of the human NK-2 receptor is given in N.P. Gerard, et al., Journal of Biological Chemistry, 265:20455-20462 (1990).

For preparation of membranes, 30 confluent roller bottle cultures are dissociated by washing each roller bottle with 10 ml of Dulbecco's phosphate buffered saline (PBS) without calcium and magnesium, followed by addition of 10 ml of enzyme-free cell dissociation solution (PBS-based, from Specialty Media, Inc.). After an additional 15 minutes, the dissociated cells are pooled and centrifuged at 1,000 RPM for 10 minutes in a clinical centrifuge. Membranes are prepared by homogenization of the cell pellets in 300 ml 50 mM Tris buffer, pH 7.4 with a Tekmar® homogenizer for 10-15 seconds, followed by centrifugation at 12,000 RPM (20,000 x g) for 30 minutes using a Beckman JA-14® rotor. The pellets are washed once using the above procedure. and the final pellets are resuspended in 100-120 ml 50 mM Tris buffer, pH 7.4, and 4 ml aliquots stored frozen at -70°C. The protein concentration of this preparation is 2 mg/ml.

For the receptor binding assay, one 4-ml aliquot of the CHO-hNK-2R membrane preparation is suspended in 40 ml of assay buffer containing 50 mM Tris, pH 7.4, 3 mM manganese chloride, 0.02% bovine serum albumin (BSA) and 4 g/ml chymostatin. A 200 μl volume of the homogenate (40 μg protein) is used per sample. The radioactive ligand is [$^{125}$I]iodohistidyl-neurokinin A (New England Nuclear, NEX-252), 2200 Ci/mmol. The ligand is prepared in assay buffer at 20 nCi per 100 μl; the final concentration in the assay is 20 pM. Non-specific binding is determined using 1 μM eledoisin. Ten concentrations of eledoisin from 0.1 to 1000 nM are used for a standard concentration-response curve.

All samples and standards are added to the incubation in 10 μl dimethylsulfoxide (DMSO) for screening (single dose) or in 5 μl DMSO for $IC_{50}$ determinations. The order of additions for incubation is 190 or 195 μl assay buffer, 200 μl homogenate, 10 or 5 μl sample in DMSO, 100 μl radioactive ligand. The samples are incubated 1 hour at room temperature and then filtered on a cell harvester through filters which had been presoaked for two hours in 50 mM Tris buffer, pH 7.7, containing 0.5% BSA. The filter is washed 3 times with approximately 3 ml of cold 50 mM Tris buffer, pH 7.7. The filter circles are then punched into 12 x 75 mm polystyrene tubes and counted in a gamma counter.

As the compounds of Formula I are effective tachykinin receptor antagonists, these compounds are of value in the treatment of a wide variety of clinical conditions which are characterized by the presence of an excess of tachykinin. Thus, the invention provides methods for the treatment or prevention of a physiological disorder associated with an excess of tachykinins, which method comprises administering to a mammal in need of said treatment an effective amount of a compound of Formula I or a pharmaceutically acceptable salt, solvate or prodrug thereof. The term "physiological disorder associated with an excess of tachykinins" encompasses those disorders associated with an inappropriate stimulation of tachykinin receptors, regardless of the actual amount of tachykinin present in the locale.

These physiological disorders may include disorders of the central nervous system such as anxiety, depression, psychosis, and schizophrenia; neurodegenerative disorders such as dementia, including senile dementia of the Alzheimer's type, Alzheimer's disease, AIDS-associated dementia, and Down's syndrome; demyelinating diseases such as multiple sclerosis and amyotrophic lateral sclerosis and other neuropathological disorders such as peripheral neuropathy, such as diabetic and chemotherapy-induced neuropathy, and post-herpetic and other neuralgias; acute and chronic obstructive airway diseases such as adult respiratory distress syndrome, bronchopneumonia, bronchospasm, chronic bronchitis, drivercough, and asthma; inflammatory diseases such as inflammatory bowel disease, psoriasis, fibrositis, osteoarthritis, and rheumatoid arthritis; disorders of the musculo-skeletal system, such as osteoporosis; allergies such as eczema and rhinitis; hypersensitivity disorders such as poison ivy; ophtalmic diseases such as conjunctivitis, vernal conjunctivitis, and the like; cutaneous diseases such as contact dermatitis, atopic dermatitis, urticaria, and other eczematoid dermatites; addiction disorders such as alcoholism; stress-related somatic disorders; reflex sympathetic dystrophy such as shoulder/hand syndrome; dysthymic disorders; adverse immunological reactions such as rejection of transplanted tissues and disorders related to immune enhancement or suppression such as systemic lupus erythematosis; gastrointestinal disorders or diseases associated with the neuronal control of viscera such as ulcerative colitis, Crohn's disease, emesis, and irritable bowel syndrome; disorders of bladder function such as bladder detrusor hyper-reflexia and incontinence; artherosclerosis; fibrosing and collagen diseases such as scleroderma and eosinophilic fascioliasis; irritative symptoms of benign prostatic hypertrophy; disorders of blood flow caused by vasodilation and vasospastic diseases such as angina, migraine, and Reynaud's disease; and pain or nociception, for example, that attributable to or associated with any of the foregoing conditions, especially the transmission of pain in migraine. For example the compounds of Formula I may suitably be used in the treatment of disorders of the central nervous system such as anxiety, psychosis, and schizophrenia; neurodegenerative disorders such as Alzheimer's disease and Down's syndrome; respiratory diseases such as bronchospasm and asthma; inflammatory diseases such as inflammatory bowel disease, osteoarthritis and rheumatoid arthritis; adverse immunological disorders such as rejection of transplanted tissues; gastrointestinal disorders and diseases such as disorders associated with the neuronal control of viscera such as ulcerative colitis, Crohn's disease, emesis, and irritable bowel syndrome; incontinence; disorders of blood flow caused by vasodilation; and pain or nociception, for example, that attributable to or associated with any of the foregoing conditions or the transmission of pain in migraine.

Many of the compounds of Formula I are selective tachykinin receptor antagonists. These compounds preferentially bind one tachykinin receptor subtype compared to other such receptors. Such compounds are especially preferred.

For example, NK-1 antagonists are most especially preferred in the treatment of pain, especially chronic pain, such as neuropathic pain, post-operative pain, and migraines, pain associated with arthritis, cancer-associated pain, chronic lower back pain, cluster headaches, herpes neuralgia, phantom limb pain, central pain, dental pain, neuropathic pain, opioid-resistant pain, visceral pain, surgical pain, bone injury pain, pain during labor and delivery, pain resulting from burns, including sunburn, post partum pain, angina pain, and genitourinary tract-related pain including cystitis.

In addition to pain, NK-1 antagonists are especially preferred in the treatment and prevention of urinary incontinence; irritative symptoms of benign prostatic hypertrophy; motility disorders of the gastrointestinal tract, such as irritable bowel syndrome; acute and chronic obstructive airway diseases, such as bronchospasm, bronchopneumonia, asthma, and adult respiratory distress syndrome; artherosclerosis; inflammatory conditions, such as inflammatory bowel disease, ulcerative colitis, Crohn's disease, rheumatoid arthritis, osteoarthritis, neurogenic inflammation, allergies, rhinitis, cough, dermatitis, urticaria, psoriasis, conjunctivitis, emesis, irritation-induced miosis; tissue transplant rejection; plasma extravasation resulting from cytokine chemotherapy and the like; spinal cord trauma; stroke; cerebral stroke (ischemia); Alzheimer's disease; Parkinson's disease; multiple sclerosis; amyotrophic lateral sclerosis; schizophrenia; anxiety; and depression.

NK-2 antagonists are especially preferred in the treatment of urinary incontinence, bronchospasm, asthma, adult respiratory distress syndrome, motility disorders of the gastrointestinal tract, such as irritable bowel syndrome, and pain.

The compounds of Formula I are usually administered in the form of pharmaceutical compositions. These compounds can be administered by a variety of routes including oral, rectal, transdermal, subcutaneous, intravenous, intramuscular, and intranasal. These compounds are effective as both injectable and oral compositions. Such compositions are prepared in a manner well known in the pharmaceutical art and comprise at least one active compound.

The present invention also includes pharmaceutical compositions which contain, as the active ingredient, the compounds of Formula I associated with pharmaceutically acceptable carriers. In making the compositions of the present invention the active ingredient is usually mixed with an excipient, diluted by an excipient or enclosed within such a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin

capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents such as talc, magnesium stearate, and mineral oil; wetting agents; emulsifying and suspending agents; preserving agents such as methyl- and propylhydroxybenzoates; sweetening agents; and flavoring agents. The compositions of the invention can be formulated so as to provide quick, sustained or delayed release of the active ingredient after administration to the patient by employing procedures known in the art.

The compositions are preferably formulated in a unit dosage form, each dosage containing from about 0.05 to about 100 mg, more usually about 1.0 to about 30 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical excipient.

The active compound is effective over a wide dosage range. For examples, dosages per day normally fall within the range of about 0.01 to about 30 mg/kg of body weight. In the treatment of adult humans, the range of about 0.1 to about 15 mg/kg/day, in single or divided dose, is especially preferred. However, it will be understood that the amount of the compound actually administered will be determined by a physician, in the light of the relevant circumstances, including the condition to be treated, the chosen route of administration, the actual compound administered, the age, weight, and response of the individual patient, and the severity of the patient's symptoms, and therefore the above dosage ranges are not intended to limit the scope of the invention in any way. In some instances dosage levels below the lower limit of the aforesaid range may be more than adequate, while in other cases still larger doses may be employed without causing any harmful side effect, provided that such larger doses are first divided into several smaller doses for administration throughout the day.

Formulation Example 1

Hard gelatin capsules containing the following ingredients are prepared:

| Ingredient | Quantity (mg/capsule) |
|---|---|
| Active Ingredient | 30.0 |
| Starch | 305.0 |
| Magnesium stearate | 5.0 |

The above ingredients are mixed and filled into hard gelatin capsules in 340 mg quantities.

Formulation Example 2

A tablet formula is prepared using the ingredients below:

| Ingredient | Quantity (mg/tablet) |
|---|---|
| Active Ingredient | 25.0 |
| Cellulose, microcrystalline | 200.0 |
| Colloidal silicon dioxide | 10.0 |
| Stearic acid | 5.0 |

The components are blended and compressed to form tablets, each weighing 240 mg.

Formulation Example 3

A dry powder inhaler formulation is prepared containing the following components:

| Ingredient | Weight % |
|---|---|
| Active Ingredient | 5 |
| Lactose | 95 |

The active mixture is mixed with the lactose and the mixture is added to a dry powder inhaling appliance.

Formulation Example 4

Tablets, each containing 30 mg of active ingredient, are prepared as follows:

| Ingredient | Quantity (mg/tablet) |
|---|---|
| Active Ingredient | 30.0 mg |
| Starch | 45.0 mg |
| Microcrystalline cellulose | 35.0 mg |
| Polyvinylpyrrolidone (as 10% solution in water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U.S. sieve. The granules so produced are dried at 50-60°C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 120 mg.

Formulation Example 5

Capsules, each containing 40 mg of medicament are made as follows:

| Ingredient | Quantity (mg/capsule) |
|---|---|
| Active Ingredient | 40.0 mg |
| Starch | 109.0 mg |
| Magnesium stearate | 1.0 mg |
| Total | 150.0 mg |

The active ingredient, cellulose, starch, and magnesium stearate are blended, passed through a No. 20 mesh U.S. sieve, and filled into hard gelatin capsules in 150 mg quantities.

Formulation Example 6

Suppositories, each containing 25 mg of active ingredient are made as follows:

| Ingredient | Amount |
|---|---|
| Active Ingredient | 25 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

Formulation Example 7

Suspensions, each containing 50 mg of medicament per 5.0 ml dose are made as follows:

| Ingredient | Amount |
|---|---|
| Active Ingredient | 50.0 mg |
| Xanthan gum | 4.0 mg |
| Sodium carboxymethyl cellulose (11%) | |
| Microcrystalline cellulose (89%) | 50.0 mg |
| Sucrose | 1.75 g |
| Sodium benzoate | 10.0 mg |
| Flavor and Color | q.v. |
| Purified water to | 5.0 ml |

The medicament, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

Formulation Example 8

Capsules, each containing 15 mg of medicament, are made as follows:

| Ingredient | Quantity (mg/capsule) |
|---|---|
| Active Ingredient | 15.0 mg |
| Starch | 407.0 mg |
| Magnesium stearate | 3.0 mg |
| Total | 425.0 mg |

The active ingredient, cellulose, starch, and magnesium stearate are blended, passed through a No. 20 mesh U.S. sieve, and filled into hard gelatin capsules in 425 mg quantities.

Formulation Example 9

An intravenous formulation may be prepared as follows:

| Ingredient | Quantity |
|---|---|
| Active Ingredient | 250.0 mg |
| Isotonic saline | 1000 ml |

Formulation Example 10

A topical formulation may be prepared as follows:

| Ingredient | Quantity |
|---|---|
| Active Ingredient | 1-10 g |
| Emulsifying Wax | 30 g |
| Liquid Paraffin | 20 g |
| White Soft Paraffin | to 100 g |

The white soft paraffin is heated until molten. The liquid paraffin and emulsifying wax are incorporated and stirred until dissolved. The active ingredient is added and stirring is continued until dispersed. The mixture is then cooled until solid.

Formulation Example 11

Sublingual or buccal tablets, each containing 10 mg of active ingredient, may be prepared as follows:

| Ingredient | Quantity Per Tablet |
|---|---|
| Active Ingredient | 10.0 mg |
| Glycerol | 210.5 mg |
| Water | 143.0 mg |
| Sodium Citrate | 4.5 mg |
| Polyvinyl Alcohol | 26.5 mg |
| Polyvinylpyrrolidone | 15.5 mg |
| Total | 410.0 mg |

The glycerol, water, sodium citrate, polyvinyl alcohol, and polyvinylpyrrolidone are admixed together by continuous stirring and maintaining the temperature at about 90°C. When the polymers have gone into solution, the solution is cooled to about 50-55°C and the medicament is slowly admixed. The homogenous mixture is poured into forms made of an inert material to produce a drug-containing diffusion matrix having a thickness of about 2-4 mm. This diffusion matrix is then cut to form individual tablets having the appropriate size.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. See. e.g., U.S. Patent 5,023,252, issued June 11, 1991, herein incorporated by reference. Such patches may be constructed for continuous, pulsatile, or on demand delivery of pharmaceutical agents.

Frequently, it will be desirable or necessary to introduce the pharmaceutical composition to the brain, either directly or indirectly. Direct techniques usually involve placement of a drug delivery catheter into the host's ventricular system to bypass the blood-brain barrier. One such implantable delivery system, used for the transport of biological factors to specific anatomical regions of the body, is described in U.S. Patent 5,011,472, issued April 30, 1991, which is herein incorporated by refernce.

Indirect techniques, which are generally preferred, usually involve formulating the compositions to provide for drug latentiation by the conversion of hydrophilic drugs into lipid-soluble drugs or prodrugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions which can transiently open the blood-brain barrier.

## Claims

1. A compound of the formula

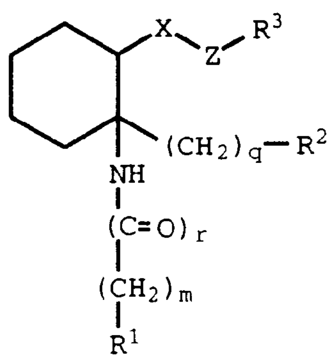

wherein:

X is -NH-, -O-, or $-S(O)_p-$,

where p is 0-2;

Z is $-(CHR^4)_j-(CHR^6)_k-$, where,

j is 0 or 1;

k is 0 or 1; and

$R^4$ and $R^6$ are independently selected from the group consisting of hydrogen and $C_1$-$C_3$ alkyl;

m and q are independently 0-3;

r is 0 or 1;

$R^3$ is phenyl, phenyl-($C_1$-$C_6$ alkylidenyl)-, $C_3$-$C_8$ cycloalkyl, $C_5$-$C_8$ cycloalkenyl, $C_1$-$C_8$ alkyl, naphthyl, $C_2$-$C_8$ alkenyl, or hydrogen;

any one of which groups except hydrogen may be substituted with one or two moieties independently selected from the group consisting of halo, $C_1$-$C_3$ alkoxy, $C_1$-$C_3$ alkylthio, nitro, trifluoromethyl, and $C_1$-$C_3$ alkyl; and

$R^2$ is phenyl, 3-indolyl, 2-indolyl, 3-benzothienyl, 3-benzofuranyl, 3-benzofuranyl, or naphthyl, said groups being optionally substituted with one to three moieties independently selected from the group consisting of $C_1$-$C_6$ alkyl, $C_1$-$C_6$ alkoxy, halo, or trifluoromethyl;

$R^1$ is trityl, phenyl, diphenylmethyl, phenoxy, phenylthio, hexamethyleneiminyl, piperazinyl, piperidinyl, pyrrolidinyl, morpholinyl, indolinyl, indolyl, benzothienyl, benzofuranyl, quinolinyl, isoquinolinyl, tetrahydropyridinyl, reduced quinolinyl, reduced isoquinolinyl, phenyl-($C_1$-$C_6$ alkylidenyl)-, phenyl-($C_1$-$C_4$ alkoxy)-, quinolinyl-($C_1$-$C_6$ alkylidenyl)-, isoquinolinyl-($C_1$-$C_6$ alkylidenyl)-, reduced quinolinyl-($C_1$-$C_6$ alkylidenyl)-, reduced isoquinolinyl-($C_1$-$C_6$ alkylidenyl)-, benzoyl-($C_1$-$C_6$ alkylidenyl)-, $C_1$-$C_4$ alkyl, or $-NH-CH_2-R^5$;

any one of which $R^1$ groups may be substituted with halo, $C_1$-$C_4$ alkyl, $C_1$-$C_4$ alkoxy, trifluoromethyl, amino, $C_1$-$C_4$ alkylamino, or di($C_1$-$C_4$ alkyl)amino;

or any one of which $R^1$ groups may be substituted with phenyl, piperazinyl, $C_3$-$C_8$ cycloalkyl, benzyl, $C_1$-$C_4$ alkyl, piperidinyl, pyridinyl, pyrimidinyl, $C_2$-$C_6$ alkanoylamino, pyrrolidinyl, $C_2$-$C_6$ alkanoyl, or $C_1$-$C_4$ alkoxycarbonyl;

any one of which groups may be substituted with halo, $C_1$-$C_4$ alkyl, $C_1$-$C_4$ alkoxy, trifluoromethyl, amino, $C_1$-$C_4$ alkylamino, di($C_1$-$C_4$ alkyl)amino, or $C_2$-$C_4$ alkanoylamino; and

$R^5$ is pyridyl, anilino-($C_1$-$C_6$ alkylidenyl)-, or anilinocarbonyl;

or a salt or solvate thereof.

2. A compound as claimed in Claim 1 wherein $R^3$ is phenyl, or phenyl-($C_1$-$C_6$ alkyl)-, optionally substituted with one or two chloro, fluoro, trifluoromethyl, methoxy, ethoxy, methyl, or ethyl groups, or a salt or solvate of said compound.

3. A compound as claimed in Claim 2 wherein $R^2$ is phenyl, 2-indolyl, 3-indolyl, 2-indolinyl, 3-indolinyl, or naphthyl optionally substituted with one or two chloro, fluoro, methyl, ethyl, methoxy, or ethoxy groups, or a salt or solvate of said compound.

4. A compound as claimed in Claim 3 wherein $R^1$ is hydrogen, phenyl, piperazinyl, piperidinyl, morpholinyl, benzofuranyl, phenyl-($C_1$-$C_4$ alkyl)-, phenyl-($C_1$-$C_4$ alkoxy)-, -NH-$CH_2$-$R^5$, any one of which groups may be substituted, or a salt or solvate of said compound.

5. A compound as claimed in Claim 4 wherein $R^1$ is hydrogen, 1-(4-phenyl)piperazinyl, 1-(4-cyclohexyl)piperazinyl, 1-(4-phenyl)piperidinyl, 1(4-cyclohexyl)piperidinyl, 1-(4-isopropyl)piperazinyl, or 1-[4-(1-piperidinyl)]piperidinyl, or a salt or solvate of said compound.

6. A pharmaceutical formulation comprising as an active ingredient a compound as claimed in any one of claims 1 to 5, associated with one or more pharmaceutically acceptable carriers, diluents, or excipients therefor.

7. A compound as claimed in any one of Claims 1 to 5 for use in the treatment or prevention of a physiological disorder associated with an excess of tachykinins.

8. A process for preparing a compound as claimed in any one of Claims 1 to 5 in which r is 1, which comprises reacting a compound of the formula

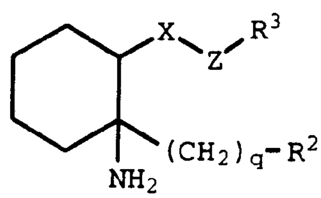

with a compound of the formula

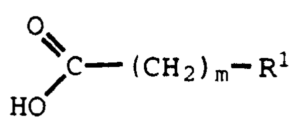

or a salt or solvate thereof, optionally in the presence of an activating agent.